(19) 

**Europäisches Patentamt**
**European Patent Office**
**Office européen des brevets**

(11) **EP 4 717 181 A1**

(12) **EUROPEAN PATENT APPLICATION**

(43) Date of publication:
**01.04.2026 Bulletin 2026/14**

(21) Application number: **24203514.5**

(22) Date of filing: **30.09.2024**

(51) International Patent Classification (IPC):
***A61B 6/46*** *(2024.01)*

(52) Cooperative Patent Classification (CPC):
**A61B 6/505; A61B 6/032; A61B 6/466;**
**A61B 6/5217; G06T 7/0012; G16H 50/20;**
G06T 2207/10081; G06T 2207/20081;
G06T 2207/20084; G06T 2207/30012

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Bonescreen GmbH**
**81671 München (DE)**

(72) Inventor: **TETTEH, Giles**
**81671 München (DE)**

(74) Representative: **Laqua, Bernd Christian Kurt**
**Wuesthoff & Wuesthoff**
**Patentanwälte und Rechtsanwalt PartG mbB**
**Schweigerstraße 2**
**81541 München (DE)**

(54) **METHOD, COMPUTING SYSTEM, COMPUTER PROGRAM PRODUCT AND COMPUTER-READABLE MEDIUM FOR VISUALIZING VOLUMETRIC BONE MINERAL DENSITY**

(57)     A method for generating a three-dimensional visualization for assessing a volumetric bone mineral density, vBMD, of a patient is provided, the method being performed by a computing system and comprising: obtaining (S1010) a computed tomography, CT, scanning image of a region of the patient; segmenting (S1020) at least one vertebra of the patient located within the obtained image; identifying (S1030), by a first artificial intelligence, AI, module, at least one segment of the at least one segmented vertebra and determine suitability for vBMD assessment of the at least one segmented vertebra based on the identified at least one segment; excluding (S1030) the at least one vertebra from the segmented image if it is determined to be unsuitable for vBMD assessment; calculating (S1050) a vBMD of a non-excluded at least one segmented vertebra; generating (S1070) a three-dimensional representation of the calculated vBMD; and visualizing (S1070) the generated three-dimensional representation on a display.

Figure 4

**Description**

FIELD OF THE INVENTION

The present invention generally relates to a method of generating a three-dimensional visualization for assessing a volumetric bone mineral density (vBMD) of a patient, an associated computing system, an associated computer program product and an associated computer readable medium.

BACKGROUND TO THE INVENTION

Measurements of bone mineral density (BMD) are useful in the detection of osteoporosis and osteopenia, in the estimation of fracture risk, and in the detection of other conditions of impaired bone health. BMD can be measured using x-ray-based methods.

One "classic" method is Dual-Energy X-ray Absorptiometry (DEXA), which measures a two-dimensional areal BMD (aBMD), expressed in mg/cm$^2$. The accuracy of DEXA can be limited by several factors, such as the size and shape of the bone and body habitus, particularly in very obese or very thin patients. Since DEXA averages aBMD over the entire area scanned, focal variations in BMD can be masked. This can be problematic in conditions like osteoporosis, where density changes may be focal.

A method less prone to these errors is quantitative computed tomography (QCT), which measures three-dimensional volumetric BMD (vBMD), expressed in mg/cm$^3$ of equivalent calcium hydroxyapatite. QCT typically involves image acquisition, definition of a region of interest (ROI) for BMD measurement, extraction of the Hounsfield Units (HU) from those regions, determining a calibration equation to convert HU into BMD, and reporting the average BMD.

For BMD measurements in QCT, the trabecular region of bone is preferred over the cortical region, especially at the spine and other bone regions. The trabecular (spongy) bone is highly vascularized, has a larger surface area compared to cortical bone, and undergoes more rapid turnover and remodeling. This makes it more sensitive to metabolic changes and interventions (e.g., treatment for osteoporosis). This sensitivity allows for earlier detection of bone loss and more responsive monitoring of treatment effects. In contrast, cortical bone is denser and has a slower metabolic turnover rate. Changes in bone density occur more slowly in cortical bone, making it less sensitive to early changes in bone mass, and less responsive to short-term interventions. Furthermore, the trabecular bone contributes significantly to the overall mechanical strength and integrity of bones, particularly at the spine. Measuring BMD in trabecular bone provides insight into the structural competence of these critical areas, which is essential for assessing fracture risk.

BMD can be measured in absolute numbers and visualized. Some known methods visualize CT HU values/density distributions with color-coded maps at various bone regions. Other known methods use voxel-based finite element (FE) visualizations based on corresponding BMD values in calibrated QCT scans. US 2015/0348259 A1 describes a method for generating and visualizing volumetric BMD from 3D image content of segmented trabecular content in line with this.

With the advantages in machine learning (ML) and computational power, semi-automated CT tools for quantitative BMD assessment (focusing on numeric values, not visualization) have been developed. Known methods of this relate to a semi-automated BMD assessment tool for the lumbar spine, using automated segmentation and ROI selection in non-fractured vertebrae at the lumbar spine.

However, the above known methods face several problems. The measurement and display of CT-based BMD values at the spine faces numerous challenges due to anatomical variations and the aging process affecting the bone.

Vertebral fractures (VFs) which increasingly occur with age, can significantly alter the anatomy and density of the affected vertebrae, leading to inaccurate BMD readings that do not reflect the true bone health of the region. Consequently, fractured vertebrae are typically manually excluded from BMD measurements. Solutions for automated detection of VFs exist, but have thus far not been combined with BMD measurement tools.

Severe degenerative changes, such as osteophytes or endplate sclerosis, can artificially increase the apparent BMD, even in the trabecular region of vertebrae in CT-based measurements, masking the presence of osteoporosis or low BMD. Therefore, severely degenerated vertebrae are also typically manually excluded from BMD measurements.

Smaller changes that may not be detected negatively influence the accuracy of measurements.

Spinal surgeries are common, especially in elderly patients. Such interventions include the placement of foreign material (e.g., cement or screws) that heavily influence the accuracy of subsequent BMD measurements. Consequently, vertebrae with foreign material are typically manually excluded from BMD measurements.

When assessing BMD at the spine, measurements are usually taken from multiple lumbar vertebrae, specifically L1 through L3 (the lumbar vertebrae are designated L1 to L5, starting at the top). This approach helps average out significant variations in bone density between individual vertebrae, providing a more accurate and reliable overall assessment of the lumbar spine's bone density. This reduces the impact of localized abnormalities or variations present in a single vertebra. However, bone density can vary significantly between different spine levels (i.e., cervical, thoracic, and lumbar vertebrae). Various investigations have found only low to moderate correlations between BMD at different spinal levels. Some of these investigations have found significant correlations between the BMD at the cervicothoracic spine and the lumbar spine, and have proposed cut-off values for the diagnosis of osteoporosis and osteopenia. However, until now, spinal BMD measurements at areas other than L1-3 cannot be used for diagnosis, as thresholds have only been established for L1-3. Any other visualization of cervicothoracic BMD may therefore be misleading to the radiologist/physician due to physiological BMD variations throughout the spine. Thus, a considerable amount of data (e.g., CT scans only containing the cervical spine) remains unused.

In summary, semi-automated BMD assessment and display of BMD with currently available methods is possible at the lumbar spine. However, all methods show two main problems:

a) Residual manual processes are required to select good measuring data. This is laborious and impractical for broader implantation into the clinical routine; and

b) There is no possibility to display BMD at the cervicothoracic spine in a clinically useful manner due to missing thresholds and inherent variations in BMD at different spinal levels.

Accordingly, there is a need for a technique that avoids one or more of the problems discussed above, or other problems.

SUMMARY OF THE INVENTION

The invention is set out in the independent claims. Preferred embodiments of the invention are set out in the dependent claims.

According to a first aspect, we describe a method for generating a three-dimensional visualization for assessing a volumetric bone mineral density (vBMD) of a patient, the method being performed by a computing system and comprising: obtaining a computed tomography (CT) scanning image of a region of the patient; segmenting at least one vertebra of the patient located within the obtained image; identifying, by a first artificial intelligence (AI) module, at least one segment of the at least one segmented vertebra and determine suitability for vBMD assessment of the at least one segmented vertebra based on the identified at least one segment; excluding the at least one vertebra from the segmented image if it is determined to be unsuitable for vBMD assessment; calculating a vBMD of a non-excluded at least one segmented vertebra; generating a three-dimensional representation of the calculated vBMD; and visualizing the generated three-dimensional representation on a display.

The obtaining of the CT scanning image may comprise obtaining and/or processing 3D image data from various sources. A communication interface may interact with a range of image sources, such as computer networks, imaging databases, imaging computers, or imaging apparatuses. In some examples, a data receiver retrieves image data from the connected source, which may include image intensities with a specific signal dynamic range. This image data may be 2D or 3D, or a combination of both should a plurality of images be received by the data receiver. Once the data is received, a data processor may prepare it for further analysis by subsequent components of the system. This may include checking the viability of the image data for further processing. By checking the viability, the possibility of bad data/images being used for the assessment of the vBMD may be reduced, thereby improving the assessment of the vBMD. In some examples, the communication interface and/or data receiver and/or data processor are part of the computing system described herein.

The segmenting may comprise segmenting subregions of each vertebra in the obtained (3D) scanning image (data).

The identifying and excluding may comprise analyzing the segmented vertebrae and their corresponding subregions to determine their suitability for vBMD calculation. Vertebrae with abnormalities and/or artifacts and/or degenerative changes that may affect the accuracy of vBMD measurements may be identified and excluded from further analysis. This may, in turn, improve the vBMD assessment. The first AI module, used for at least the identification step, may, in some examples, comprise any suitable type of neural network or other machine learning method.

The generating and visualizing may comprise generating a three-dimensional representation of the calculated vBMD values, and visualizing these on a display to a medical professional to facilitate medical diagnosis. The visualization of the three-dimensional representation of the calculated vBMD values may be in any suitable manner that allows for a medical professional, or user of the method, to easily identify areas of interest and/or concern in the vBMD assessment, thereby improving assessment of the vBMD and aiding diagnosis of the patient.

In some examples, the calculated vBMD is a cervicothoracic vBMD of the non-excluded at least one segmented vertebra, wherein the method further comprises converting (S1060) the calculated cervicothoracic vBMD to a lumbar-equivalent vBMD based on at least one predetermined conversion factor, wherein the three-dimensional representation is generated from the converted vBMD.

The converting may comprise utilizing, preferably linear, mapping parameters to convert cervicothoracic vBMD values into lumbar-equivalent vBMD values, although the skilled person understands non-linear mapping parameters may additionally or alternatively be used.

In some examples, the segmentation is performed using a shape (or "shape-based") segmentation method comprising an active shape model and/or a statistical shape model. This may involve leveraging prior knowledge of the vertebral anatomy and shape variations and guiding a template shape of subregions for every vertebral level based on user-defined objective functions for accurate shape delineation. This, in turn, improves the segmentation step of the method, and allows for a more accurate vBMD measurement.

In some examples, the segmentation is performed using a second AI module, wherein the second AI module is trained based on a plurality of CT scanning images different from the obtained scanning image, wherein the plurality of CT scanning images comprise various segmentations of at least one vertebra. This may allow for an improved segmentation and a more accurate vBMD measurement. This may additionally or alternatively allow for the region of interest and, in the later steps of the method, the extent of the deformity to be ascertained. The second AI module may, in some examples, comprise any suitable type of neural network or other machine learning method.

In some examples, segmentation is performed on the plurality of CT scanning images based on the shape segmentation method to generate a plurality of labelled segmented scanning images, wherein the second AI module is trained based on the plurality of labelled segmented scanning images. The above-mentioned shape-based segmentation methods, based on e.g. a universal shape-template, may generate segmentation results that can be used at a later point in time to create or augment the training data for the learning-based approach using the second AI module, resulting in effective learning and thereby leading to a more accurate segmentation process, and a more accurate vBMD measurement.

In some examples, the segmentation step is configured to output the segmentation as a mask of the segmented image configured to delineate a structure and/or a boundary of the at least one vertebra. This may allow for a more accurate segmentation process, and a more accurate vBMD measurement.

In some examples, the segmentation comprises delineation of the at least one vertebra, in particular wherein at least one of the following regions is delineated:

- vertebral body;
- vertebral arch;
- spinous process;
- transverse process;
- superior articular process;
- inferior articular process;
- cortex of the vertebral body; and
- a trabecular component of the vertebral body.

This may allow for a more detailed segmentation process, thereby making the subsequent steps of the method easier, and a more accurate and detailed vBMD assessment. The skilled person understands that the above list is not a comprehensive list, and that any suitable region may be delineated. In some examples, a user of the method may be able to choose, via a communication interface, the region(s) to be delineated in order to focus computational resources on a particular area/region of concern/interest. Additionally or alternatively, the region(s) to be delineated may be predetermined by the method.

In some examples, the first AI module is trained to perform the exclusion based on a plurality of CT scanning images comprising segmented vertebrae different from the segmented scanning image, wherein a subset of the plurality of CT scanning images comprise at least one vertebra that is considered to be unsuitable for vBMD assessment. This may likewise lead to a more accurate vBMD measurement.

In some examples, a vertebra is determined to be unsuitable for vBMD assessment in the case of the presence of at least one of:

- an osteophyte formation on the at least one vertebra;
- a sclerotic lesion on the at least one vertebra;
- a fracture of the at least one vertebra;
- a metal implant in the at least one vertebra; and
- cement in and/or on the at least one vertebra.

This may allow for unsuitable segments and/or vertebrae to be removed from the remainder of the method, thereby leading to a more accurate vBMD measurement and assessment of the "healthy" segments and/or vertebrae. The skilled person understands that the above list is not a comprehensive list, and that any other suitable parameter may be used to determine the (un)suitability of a vertebra. In some examples, a user of the method may be able to choose, via a communication interface, the parameter(s) to be taken into account during the determination process, thereby allowing for computational resources to be focused. This may also allow for the user to discard anomalies that do not (substantially) affect the vBMD in the region being assessed. Additionally or alternatively, the parameter(s) may be predetermined by the method.

In some examples, the first AI module is a neural network and comprises at least one shared layer configured to extract common features from the segmented image, and at least one task-specific adapter in a layer subsequent to the at least one shared layer, wherein the at least one task-specific adapter is configured to analyze task-specific features. In particular, the task-specific features may be associated with at least one of the following tasks:

- fracture detection;
- degeneration assessment; and
- malignancy identification.

This may allow for unsuitable segments and/or vertebrae to be removed from the remainder of the method, thereby leading to an improved vBMD assessment and calculation. The skilled person understands that the above list is not a comprehensive list, and that any other suitable feature may be analyzed. In some examples, a user of the method may be able to choose, via a communication interface, the task-specific feature(s) to be analyzed in order to focus computational resources on a particular area/region of concern/interest. Additionally or alternatively, the task-specific feature(s) to be analyzed may be predetermined by the method.

In some examples, the first AI module is trained based on a plurality of segmented CT scanning images different from the segmented CT scanning image output in the segmentation step, and is trained to output an indication that the at least one vertebra from the segmented image is unsuitable for vBMD assessment based on at least one of the task-specific features. This may also lead to a more accurate vBMD measurement.

In some examples, the extracted common features from the segmented image relate to the at least one task-specific adapter, in particular wherein the common features are associated with at least one of:

- an anatomical structure;
- a texture of the segmented image; and
- a spatial relationship within the at least one vertebra.

This may allow for suitable common features to be extracted, thereby allowing for a more accurate vBMD assessment. The skilled person understands that the above list is not a comprehensive list, and that any suitable common feature may be extracted. In some examples, a user of the method may be able to choose, via a communication interface, the common feature(s) to be extracted in order to focus computational resources on a particular area/region of concern/interest. Additionally or alternatively, the common feature(s) to be extracted may be predetermined by the method.

In some examples, the at least one task-specific adapter is trained based on the extracted common feature(s) output from the at least one shared layer to extract a characteristic of the at least one vertebra related to its specific task, and is trained to output the indication that the at least one vertebra from the segmented image is unsuitable for vBMD assessment based on the extracted characteristic. This may likewise lead to a more accurate vBMD measurement.

In some examples, the method further comprises: calibrating the segmented image using a known calibration curve based on a known metadata parameter of the segmented image, wherein, in particular, the calibration curve is a linear calibration curve which comprises a known calibration slope and intercept based on the known metadata parameter. This may allow for verification of consistency with the segmented CT image and/or validating the segmented image against predefined acceptable ranges. This may reduce the possibility of bad data/images being used for the determination and assessment of the vBMD, thereby improving the determination of the vBMD. The metadata parameter may be any suitable metadata parameter such as, for example, CT scanner model, acquisition parameters, kVp setting, age of the patient, positioning of the patient, gender of the patient, cervical vertebrae levels, or any other suitable metadata parameter. Additionally or alternatively, the calibration may be based on one of the following methods: (1) phantom-based calibration if there is a phantom within the obtained scanning image and/or the segmented image; (2) internal calibration based on tissue references; and (3) internal calibration based on tissue references and/or scanner metadata and/or patient characteristics. In some examples, calibration may additionally or alternatively be carried out as described in patent application EP 24203433.8, where such calibration is sometimes also referred to as "adjustment".

In some examples, the computing system uses a database query module and a conversion module, wherein: the database query module is configured to communicate with a calibration database to retrieve a relevant calibration parameter based on the known metadata parameter; the conversion module is configured to generate the linear calibration curve based on the retrieved calibration parameter; and wherein the calibration step further comprises applying the linear calibration curve to the segmented image. The calibration step may calibrate the segmented image using a linear calibration curve with the known calibration slope and intercept of the metadata parameter using, for example, a database query module and a conversion module. The database query module may interact with a calibration database to retrieve the appropriate calibration parameters for the metadata parameter. If available, the query module also may retrieve the calibration parameters based on the CT scanner model and/or an acquisition parameter and/or kVp setting of the CT scanner and/or the age of the patient and/or the positioning of the patient and/or the gender of the patient and/or cervical vertebrae levels. Error checks may then performed to ensure the accuracy and integrity of the retrieved parameters, such as verifying consistency with the segmented image and validating against predefined acceptable ranges. The conversion module may generate a linear calibration curve using the retrieved calibration parameter. This curve may then be applied to the segmented image to obtain a calibrated equivalent of the segmented image. In some examples, this calibrated equivalent may then be used for the calculation, conversion, generation and visualization steps of the method.

In some examples, the conversion step further comprises: retrieving, from a database, at least one mapping parameter reflecting the at least one predetermined conversion factor, wherein the database comprises information relating to a relationship between a vBMD value of a cervicothoracic vertebra and a corresponding lumbar-equivalent vBMD value; and performing the conversion step based on the retrieved at least one mapping parameter. The at least one mapping parameter may be specific to a population-based equation factor, wherein, in particular, the at least one population-based equation factor may be based on at least one of:

- a cervicothoracic vertebra level of the patient;
- an age of the patient; and
- a gender of the patient.

This may allow for a more accurate and personalized assessment of the vBMD of the patient. The skilled person understands that the above list is not a comprehensive list, and that any suitable population-based equation factor may be used. In some examples, a user of the method may be able to choose, via a communication interface, the population-based equation factor(s) to be used in order to focus computational resources on a particular area/region of concern/interest. Additionally or alternatively, the population-based equation factor(s) to be used may be predetermined by the method.

In some examples, the three-dimensional representation of the calculated vBMD comprises a color-coded heat map of the calculated vBMD, wherein, in particular, at least one color of the color-coded heat map is based on patient information and/or a clinical guideline. Alternatively, the skilled person understands that any other suitable representation may be used that allows a user of the method to easily identify areas of interest and/or concern in the vBMD assessment. This may allow for a medical professional to easily and quickly identify a potential region of concern.

In some examples, the method is configured to visualize a vBMD of an entire spine of the patient. This may allow for a more comprehensive vBMD assessment by a medical profession, and a more comprehensive calculation of vBMD values of the patient.

According to a second aspect, we describe a computing system comprising means for carrying out the method of the first aspect. The computing system may comprise at least one processor and at least one memory, the at least one memory containing instructions executable by the at least one processor such that the computing system is operable to carry out the method according to the first aspect.

According to a third aspect, we describe a computer program product comprising instructions which, when the program is executed by a computing system, cause the computing system to carry out the method of the first aspect. The computer program product comprises program code portions for performing the method of the first aspect when the computer program product is executed on a computing system (e.g., a computing system as defined above).

According to a fourth aspect, we describe a computer-readable medium having stored thereon the computer program product of the third aspect. The computer-readable medium may be a semiconductor memory, DVD, CD-ROM, and so on.

In view of the above, the invention, as described herein, comprises a method, an apparatus, a computing system, a computer program product, and a computer-readable medium for 3D visualization for assessing a vBMD using calibrated CT scans through an approach that eliminates the need for manual ROI selection by using artificial intelligence (AI) modules for spine segmentation, endplate erosion, and the detection and exclusion of vertebrae unsuitable of vBMD assessment. It further allows the display of vBMD at the entire spine based on conversion of cervicothoracic vBMD to lumbar-equivalent vBMD values that allow the display and visualization of vBMD according to the current diagnostic guidelines and thresholds.

Any of the aspects above, in addition to their technical effects outlined above, may have one or more of the following advantages, particularly with respect to the conventional techniques of the prior art described above:

•    Vertebrae-specific conversion of cervicothoracic vBMD to lumbar-equivalent vBMD values based on conversion factors. This allows for:

- ◦ Dynamic inclusion of various regions, depending on the scan and pathology; and
- ◦ Better visual evaluation and depiction of focal variations and pathologies.

- ML-based automated exclusion of vertebrae that are not suitable for vBMD measurements, such as degenerated vertebrae, vertebrae with foreign material, etc. This allows for:

  - ◦ More reliable vBMD-assessment;
  - ◦ Better longitudinal evaluation through exclusion of new pathologies;
  - ◦ The method to be less reader-dependent;
  - ◦ Increased sensitivity and specificity in automated solutions due to such exclusions; and
  - ◦ Increased reproducibility of automated vBMD measurements due to such exclusions.

It is clear to a person skilled in the art that certain features of the method set forth herein may be implemented under use of hardware circuits, software means, or a combination thereof. Even if some of the aspects described above have been described in reference to the method, these aspects may also apply to an apparatus or computing system configured to carry out the method described herein.

BRIEF DESCRIPTION OF THE DRAWINGS

The aspects of the invention will now be further described, by way of example only, with reference to the accompanying figures, wherein like reference numerals refer to like parts, in which:

Figure 1a    shows an exemplary composition of a computing system for visualizing a vBMD of a patient according to example implementations as described herein;

Figure 1b    shows a block diagram illustrating a modular composition of a computing system for visualizing a vBMD of a patient according to example implementations as described herein;

Figure 2    shows a flow diagram of a method of segmenting at least one vertebra according to example implementations as described herein;

Figure 3    shows a diagram of a method of identifying at least one segment of the at least one segmented vertebra and the determination of suitability for vBMD assessment according to example implementations as described herein;

Figure 4    shows a flow chart of a method for visualizing a vBMD of a patient according to example implementations as described herein;

Figure 5    shows a flow chart of calculating a cervicothoracic vBMD and converting the cervicothoracic vBMD to a lumbar-equivalent vBMD according to example implementations as described herein;

Figure 6    shows a visual representation of a CT scan with and without the herein-described method according to example implementations as described herein; and

Figure 7    shows a schematic illustration of a vertebra with reference to example implementations as described herein.

DETAILED DESCRIPTION OF EMBODIMENTS

In the following description, for purposes of explanation and not limitation, specific details are set forth in order to provide a thorough understanding of the present disclosure. It will be apparent to one skilled in the art that the present disclosure may be practiced in other embodiments that depart from these specific details.

Those skilled in the art will further appreciate that the steps, services and functions explained herein below may be implemented using individual hardware circuitry, using software functioning in conjunction with a programmed microprocessor or general purpose computer. It will also be appreciated that when the present disclosure is described in terms of a method, it may also be embodied in one or more processors and one or more memories coupled to the one or more processors, wherein the one or more memories are encoded with one or more programs that perform the steps, services and functions disclosed herein when executed by the one or more processors.

The embodiments, as described herein, allow for 3D visualization of vBMD in the spine and enables accurate assessment and visualization of vBMD of not only lumbar vertebrae, but of the entire spine including the cervicothoracic region. This may improve the diagnosis and treatment of bone mineral density-related conditions, even in CT scans with a partial field-of-view, such as those obtained from trauma cases. It may also eliminate the need for dedicated scans, thereby reducing additional radiation exposure.

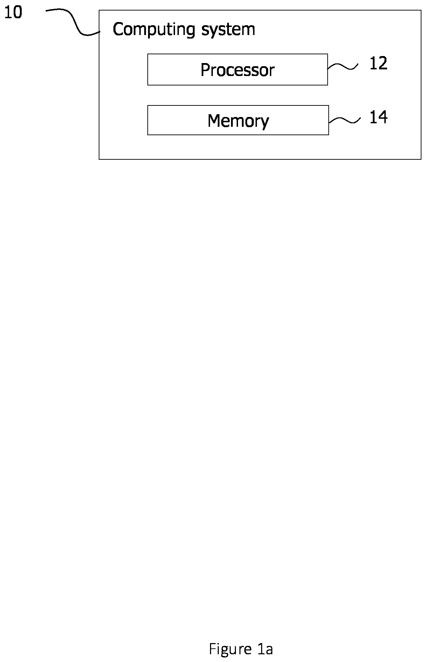

Figure 1a shows an exemplary composition of a computing system for visualizing a vBMD of a patient according to example implementations as described herein.

The computing system 10, in this example, comprises at least one processor 12 and at least one memory 14, wherein the at least one memory 14 contains instructions executable by the at least one processor 12 such that the computing unit 10 is operable to carry out the method steps described herein.

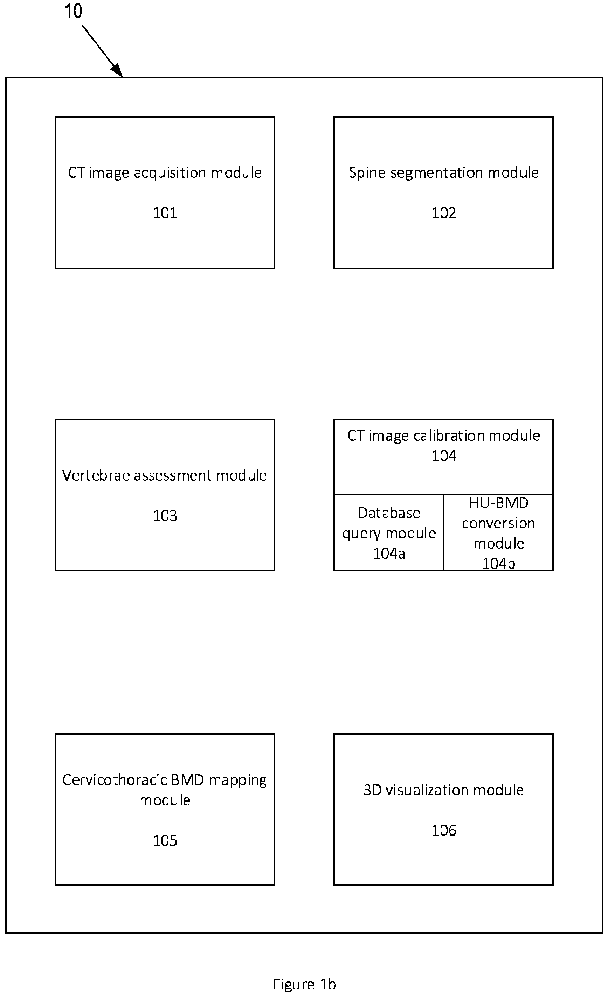

Figure 1b shows an exemplary block diagram illustrating a modular composition of a computing system for visualizing a vBMD of a patient according to example implementations as described herein.

The CT image acquirer 101 may obtain and process 3D image data from various sources. This source may be a source external to the computing system 10 described herein. A communication interface of the computing system 10 and/or CT image acquirer 101 can interact with a range of image sources, such as, for example, computer networks, imaging databases, imaging computers, or imaging apparatuses. The image source can be any suitable source as long as it is capable of transmitting a CT scanning image to the computing system 10 and/or CT image acquirer 101. The CT image acquirer 101 may, in some examples, further comprise a data receiver which is configured to retrieve 2D and/or 3D image data from the connected source, which may include image intensities with a specific signal dynamic range. The use of a specific signal dynamic range may allow for the checking of the received 2D and/or 3D image data to ensure that said data is obtained from a CT scanner that adheres to the known convention of air having a HU value of -1000 HU, and water having a HU value of 0 HU. That is to say, this ensures that the obtained data is not obtained from a faulty CT scanner and/or obtained from an MRI scanner or the like and/or that the obtained image data has not been corrupted or altered in any way. In some examples, there may be no data receiver.

Once the data is received, a data processor coupled, or integral, to the CT image acquirer 101 may prepare said data for further analysis by subsequent components of the system. This preparation may, in some examples, include checking the viability of the image data for further processing. If the data is not suitable, the data may not be used in the further steps of the method. Additionally or alternatively, a user of the method and system 10 may be alerted to the presence of bad data. In some examples, there may be no data processor.

The Spine Segmentation Module 102 may be configured to process two-dimensional (2D) and/or three-dimensional (3D) image data of a subject (patient). The module 102 may, in some examples, comprise a segmentation unit configured to segment subregions of each vertebra in the 2D and/or 3D image data obtained by the CT image acquirer 101. The segmentation of the vertebrae may comprise the delineation of a selection, or an expansion, of at least one of the following regions:

- the vertebral body;
- the vertebral arch;
- the spinous process;
- the transverse processes;
- the superior articular process;
- the inferior articular processes;
- the cortex of the vertebral body; and
- the trabecular compartment of the vertebral body.

The skilled person understands that the above is not an exhaustive list, and that any other suitable region may be delineated. Indeed, although vertebrae are disclosed herein, the skilled person understands that the system 10 and method may be applied to any other suitable area of the patient where the vBMD is to be calculated.

The above-described delineation may encompass additional or fewer vertebral components as required for the specific application of the present invention. The module 102 may be configured to output the segmented subregions as one or more sets of (3D) voxels corresponding to individual vertebrae and their respective components, i.e., the regions described above, with the trabecular compartment of the vertebral body being of particular interest for subsequent vBMD assessment and analysis in the method described herein. One or more of the above regions may be used. In the case of multiple regions being used, each region may have a unique label.

In one embodiment, the segmentation may be performed using shape-based segmentation methods such as, for example, active shape models (ASMs) and statistical shape models (SSMs). This may involve leveraging prior knowledge of the vertebral anatomy and shape variations, and guiding a template shape of subregions for every vertebral level based on user-defined objective functions for accurate shape delineation. The objective function may be, for example, Euclidean distances between the observed image data and the shape model, the negative of the gradient magnitude at landmarks, or any other suitable objective function. In some examples, the segmentation may be at least partially based on the voxels output by the Spine Segmentation Module 102.

In a further embodiment, machine learning techniques may be employed to delineate the various above-mentioned components of the vertebrae. This embodiment may utilize, for example, deep learning algorithms, such as convolutional neural networks (CNNs), trained on a plurality of datasets comprising annotated 3D images of vertebrae.

In another embodiment, the module may employ a combination of shape-based and learning-based approaches. The shape-based segmentation methods, e.g., based on a universal shape-template, generate segmentation results that can then be used to create or augment the training data for the learning-based approach, resulting in effective learning.

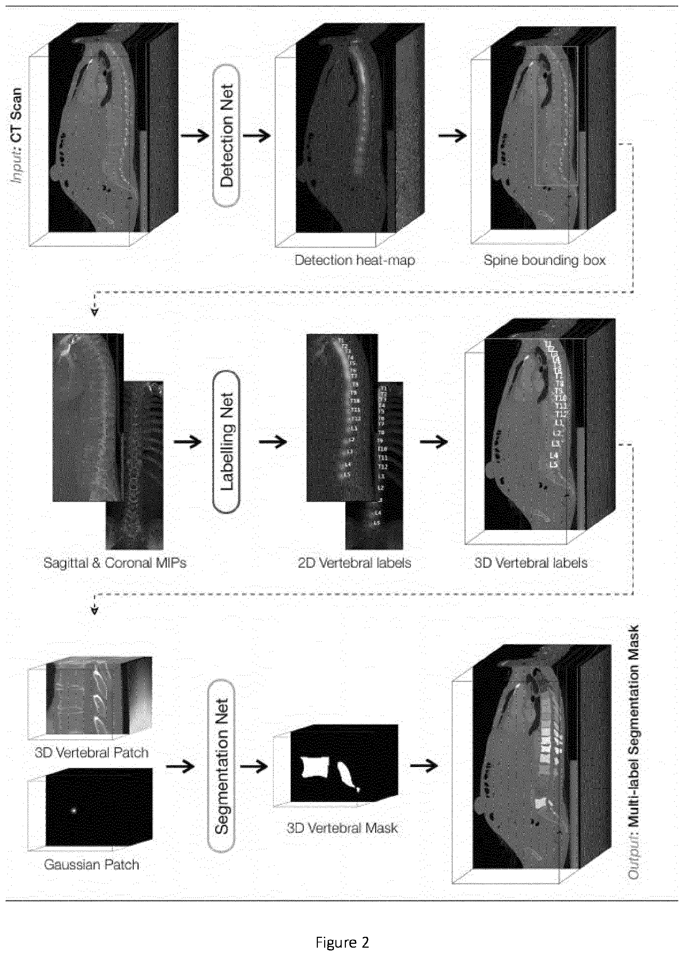

Such machine learning techniques may be undertaken by a first artificial intelligence (AI) module. This module is described below in further detail in relation to Figure 2.

The Vertebra Assessment Module 103 may be responsible for identifying and excluding vertebrae that are unsuitable for the volumetric bone mineral density (vBMD) assessment. This component may analyze the segmented vertebrae and their corresponding subregions, as obtained from the Spine Segmentation Module 102, to determine their suitability for vBMD calculation. Vertebrae with abnormalities and/or artifacts and/or degenerative changes that may affect the accuracy of vBMD measurements may be identified and excluded from further analysis. Examples of such vertebrae include, but are not limited to, vertebrae with severe osteophyte formations and/or sclerotic lesions and/or fractures and/or metal implants, as well as vertebrae with substantial degenerative disc disease or any other suitable pathology that could impact the vBMD assessment. The Vertebrae Assessment Module 103 may comprise of a convolutional neural network (CNN) with multiple task-specific adapters (e.g., in the last layer), with the adapters, in some examples, being configured for multi-task learning of vertebral abnormalities. Each adapter may correspond to a distinct task, such as fracture detection and/or degeneration assessment and/or malignancy identification. The shared CNN layers may extract common features, while the adapters may analyze task-specific features. Additionally, rule-based methods may detect artifacts, such as metal implants and/or cement. For this, the output of the Spine Segmentation Module 102, in, in this example, the form of mask may be utilized to ascertain the region of interest and the extant of the deformity. Other embodiments of this module may include any data-driven, learning models trained in a supervised, semi-supervised, and/or unsupervised manner.

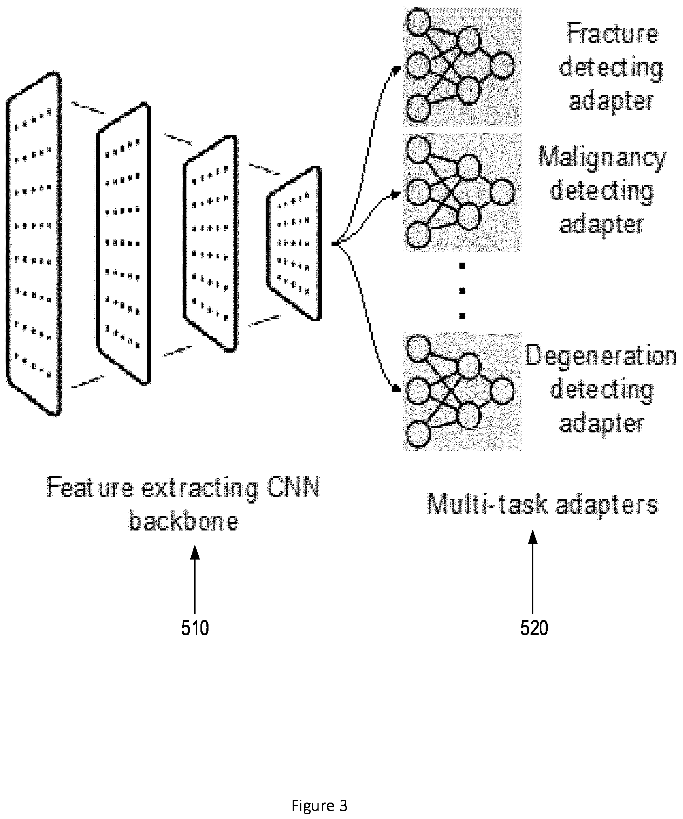

Such a CNN, as described in relation to the Vertebra Assessment Module 103, may be referred to as the second AI module herein, which is described below in further detail in relation to Figure 3.

The CT Calibration Module 104 may be responsible for calculating the calibrated CT image data using a linear HU-BMD conversion curve with known conversion slope and intercept values specific to the scanner characteristics. The module may comprise a database query module 104a and a (HU-to-BMD) conversion module 104b. The database query module 104a may interact with a curated conversion database to retrieve the appropriate conversion parameters based on scan metadata, such as the scanner model and/or acquisition parameters and/or kVp setting. Additionally or alternatively, the calibration may be based on one of the following methods: (1) phantom-based calibration if there is a phantom within the obtained scanning image and/or the segmented image; (2) internal calibration based on tissue references; and (3) internal calibration based on tissue references and/or scanner metadata and/or patient characteristics. In some examples, calibration may additionally or alternatively be carried out as described in patent application EP 24203433.8, where such calibration is sometimes also referred to as "adjustment".

Error checks may be performed to ensure the accuracy and integrity of the retrieved parameters. Such checks may comprise verifying consistency with the input CT image and validating against predefined acceptable ranges. The verification and/or validation may be based on known ranges of tissue densities within specific regions of interest. For example, for a 120kVp scanner in a (v)BMD space, fat should ideally have a density of $-80 \pm 50$ HU, and muscle should have ideally a density $30 \pm 50$ HU. The HU-to-BMD conversion module 104b may then utilize the retrieved slope and intercept values to generate a linear calibration curve and map the CT scan to its calibrated equivalent. Preferably, only the regions of the CT scan based on the vertebral delineation provided by the Spine Segmentation Module 102 and the non-excluded vertebrae provided by the Vertebra Assessment Module 103 may be used by the CT Calibration Module 104.

The Cervicothoracic BMD Mapping Module 105 may be configured to establish at least one linear mapping parameter (reflecting the at least one predetermined conversion factor, as referred to herein) between cervicothoracic vertebrae and lumbar-equivalent bone mineral density (BMD) values. This module may access a database comprising mapping parameters that have been pre-computed based on the patient metadata and/or a population-based dataset, wherein the parameters may be specific to various cervicothoracic vertebrae levels and/or patient age, and/or gender. The parameter relating to the cervicothoracic vertebrae levels may mean in this example that, if the vBMD of the C5 vertebra is to be displayed, the parameter may relate to a linear regression relating to the C5 vertebra. This database may be generated over a diverse population of patient data. Additionally or alternatively, this database may comprise information relating to the obtained scanning image such as the scanning region and/or the field of view of the obtained scanning image. Subsequently, the mapping parameters may be derived from this data by analyzing the relationships between the vBMD values of cervicothoracic vertebrae and their corresponding lumbar-equivalent values. The Cervicothoracic BMD Mapping Module 105 may utilize these linear mapping parameters to convert cervicothoracic vBMD values in the segmented image, which are optionally calibrated by the CT Calibration Module 104, into lumbar-equivalent vBMD values. The clinical thresholds defined for lumbar vertebrae can be applied to the cervicothoracic region, enabling the performance of a thorough vBMD visualization and analysis of the entire spine. In some examples, the clinal thresholds are applied to the cervicothoracic region after the conversion of the cervicothoracic vBMD to the lumbar-equivalent vBMD. In particular, the mapping parameters may be contained in a table, such as the exemplary conversion table shown below:

|     | $r^2$ | Linear Equation | Threshold Osteopenia | Threshold Osteoporosis |
|-----|-------|-----------------|----------------------|------------------------|
| C2  | 0,5772 | 1.253*x + 90.15 | 240,5 | 190,4 |
| C3  | 0,6282 | 1.266*x + 100.7 | 252,6 | 202,0 |
| C4  | 0,6434 | 1.350*x + 101.2 | 263,2 | 209,2 |
| C5  | 0,672  | 1.229*x + 99.36 | 246,8 | 197,7 |
| C6  | 0,7504 | 1.138*x +80.98  | 217,5 | 172,0 |
| C7  | 0,688  | 0.9047*x + 80.80 | 189,4 | 153,2 |
| T1  | 0,7309 | 0.8606*x + 54.50 | 157,8 | 123,3 |
| T2  | 0,7698 | 0.8758*x + 48.93 | 154,0 | 119,0 |
| T3  | 0,7449 | 0.8591*x + 39.86 | 143,0 | 108,6 |
| T4  | 0,7605 | 0.8295*x + 36.64 | 136,2 | 103,0 |
| T5  | 0,7784 | 0.8132*x + 34.69 | 132,3 | 99,7 |
| T6  | 0,7805 | 0.8190*x + 31.52 | 129,8 | 97,0 |
| T7  | 0,7974 | 0.7929*x + 32.59 | 127,7 | 96,0 |
| T8  | 0,8266 | 0.8113*x + 29.22 | 126,6 | 94,1 |
| T9  | 0,8307 | 0.8357*x + 28.50 | 128,8 | 95,4 |
| T10 | 0,874  | 0.9000*x + 23.01 | 131,0 | 95,0 |
| T11 | 0,9069 | 0.9102*x + 18.62 | 127,8 | 91,4 |
| T12 | 0,9268 | 0.9303*x + 9.346 | 121,0 | 83,8 |

The above exemplary conversion table may be used for converting the cervicothoracic vBMD to a lumbar equivalent vBMD. In particular, the $r^2$ value may relate to the correlation between the spine level and the lumbar spine. That is to say, the C2 vertebra, for example, has a correlation to the averaged lumbar spine of 0.5772. This may be referred to as the linear mapping parameter herein. An example of such conversion for the C2 vertebra is shown below:

$$x_{lumbar(L1-L3)} = \frac{(Y_{c2} - 90.15)}{1.253}$$

In the above, $x_{lumbar(L1-L3)}$ signifies the lumbar equivalent vBMD for the L1-L3 vertebrae, and $Y_{c2}$ is the measured cervicothoracic vBMD for the C2 vertebra. In particular, it can be seen that the above equation is a rearrangement of the "Linear Equation" in the above table. The skilled person understands that, due to this equation rearrangement, the lumbar-equivalent vBMD for any segmented non-lumbar vertebra can be calculated. Furthermore, should the calculated lumbar-equivalent vBMD exceed either of the thresholds in the above table, this may be indicated to a user of the method via the heatmap and/or visualization described herein in any suitable manner as a sign of the measured vertebra having one of these conditions. Alternatively, thresholds relating to clinical guidelines for the lumbar spine may be used. For these thresholds, the limits may be osteopenia may be indicated for values $\leq 120$ and for osteoporosis $\leq 80$, although the skilled person understands that other thresholds may be used.

In the following, a study set is shown, on which the above conversion table may be based in this example.

| Study Set | |
|-----------|--|
| **Patients** | |
| No. of patients | 260 |
| No. of women | 105 |
| Age (in years)[†] | 59.7 $\pm$ 18.3 |

(continued)

| Imaging | |
|---|---|
| No. of scans | 260 |
| No. of cervical spines | 212 |
| No. of vertebrae | 4874 |
| No. of contrast enhanced scans | 46 |
| No. of fractures (Genant grade 1-3) | 158 |
| No. of vertebrae (moderate to severe degenerative changes) | 530 |
| No. of patients aged <50 | 73 (21*) |
| No. of patients aged 50-59 | 49 (20*) |
| No. of patients aged 60-69 | 63 (23*) |
| No. of patients aged >70 | 75 (41*) |
| **Scanner** | |
| Philips Iqon | 29 |
| Philips Brilliance 64 | 3 |
| Philips iCT | 26 |
| Siemens Definition AS+ | 85 |
| Siemens Definition AS | 57 |
| Siemens Sensation Cardiac 64 | 11 |
| Siemens Biograph 128 | 23 |
| Siemens Biograph 64 | 26 |

It will be understood that the above study set is merely an example, and the skilled person understands that any suitable number of patients with various spinal defects and/or deformities, as well as any suitable number and types of CT scanner, may be used in order to establish such a study set. Using a study set, where the cervicothoracic vBMD and lumbar-equivalent vBMDs of patients are known, may allow for the establishment of a conversion table as exemplified above.

In some examples, there is no such conversion step at all. In such a scenario, the cervicothoracic vBMD may be measured directly, with the result of this direct measurement being generated and visualized by the 3D Visualization Module 106. The thresholds in the direct measurement may be based on clinical guidelines, with the three-dimensional representation of the vBMD values being generated and visualized based on the below description of the 3D Visualization Module 106.

The 3D Visualization Module 106 may be responsible for generating a three-dimensional representation of the calculated (and optionally converted) vBMD values. This module 106 may process the vBMD data to create a 3D model that visually displays variations in bone density through a color-coded heat map, e.g., with color codes chosen according to patient information and/or clinical guidelines. The heat map facilitates enhanced diagnostic assessment by highlighting areas of differing bone density, enabling practitioners to easily identify regions of concern. In one embodiment, the 3D visualization may be a voxelized representation, while further embodiments can additionally or alternatively include other visualizations of (3D) data, such as dense point clouds, polygonal meshes, or any other suitable (3D) rendering technique. Optionally, the 3D Visualization Module 106 can be adapted to be integrated with existing image viewers on Picture Archiving and Communication Systems (PACS), providing a convenient and efficient solution for clinical evaluation and diagnosis. Additionally or alternatively, a two-dimensional representation of the vBMD values may be generated and visualized by the 3D Visualization Module 106.

Figure 2 shows a flow diagram of a method of segmenting at least one vertebra according to example implementations as described herein. Especially, the embodiments of the Spine Segmentation Module 102 where machine learning techniques are employed to delineate the components of the vertebrae are described below.

The training process for the machine learning technique, in this example, may follow a multi-stage approach to achieve precise vertebrae segmentation.

In a first stage, a Fully Convolutional Network (FCN) may be used for localization. An FCN may be used to localize the spine within 2D and/or 3D medical images. An FCN differs from traditional convolutional networks because it replaces the fully connected layers with convolutional layers, allowing for voxel-wise predictions and image segmentation. In this example, the FCN may operate at a coarse resolution of 5mm to quickly locate the spine region, although the skilled person understands that any suitable resolution may be used. The input data of the first stage preferably comprises 3D medical images such as, for example, CT scans, while the output is preferably a probability map that highlights the spine's location.

The second stage of the machine learning technique, in this example, relates to Vertebrae Labeling. This may involve a second FCN that labels individual vertebrae. This network may be trained on a higher-resolution dataset such as, for example, a resolution of 2mm to capture more detailed features of each vertebra, although the skilled person understands that any suitable resolution may be used. Here, the inputs may preferably be cropped and rescaled 3D images of the spine based on the output of the first stage, and the outputs may preferably be labeled probability maps that indicate the locations of specific vertebrae. This can be equivalent to a 3D Gaussian map.

In a third stage, after labelling, the image may preferably be cropped into vertebral patches based on the vertebral location to isolate individual vertebrae for detailed segmentation. A further FCN may be employed for this stage, in this example, wherein this further FCN may work on patches of 3D images at 1mm resolution, although the skilled person understands that any suitable resolution may be used. This FCN may be trained to segment the vertebrae boundaries accurately. The inputs of the third stage may preferably be vertebral patches based on the output of the second stage, while the outputs may preferably be segmentation masks, which define and/or delineate the structure and boundaries of each vertebra.

The skilled person understands that some of the above stages may share a single FCN, and/or that not all of the above stages may be required in order to output the segmentation mask.

For the training of the above method, CT images with two types of annotations may be used. The first type preferably relates to 3D points denoting the x, y, and z location of the centroid of the vertebral body within the CT images, and the second type preferably relates to discrete, voxel-level, multi-label segmentation masks of the vertebra(e) within the CT images. Although CT images are mentioned, it is to be understood that any suitable type of image may be used. Additionally, the skilled person understands that the CT images may be annotated with any suitable type of annotation that allows for the training described herein in relation to the segmentation to be undertaken.

For the training of the first stage, a spinal heatmap may be generated by placing a Gaussian (with a high standard deviation) at every vertebra within the CT images. If an image is of size H*W*D, (H:height, W:width, D: depth), the output may also be of the same size H*W*D.

For the training of the second stage a heatmap per vertebra may be generated. That is to say, an output of the second stage may be of the shape H*W*D*N, where N is the number of vertebrae and each H*W*D image consists of one Gaussian placed at the corresponding vertebra.

For the training of the third stage, segmentation masks may be used, wherein the output may be the same shape as the input.

The above training is shown in more detail in figure 2.

In addition, each stage may be trained using, preferably, fully supervised learning, with the following voxel-based loss functions being applied at each stage:

For localizing the spine in the first stage, an L2 loss (mean squared error) may be used. This loss function penalizes the squared difFerences between the predicted and actual voxel values, making it suitable for regression tasks like determining the general location of the spine in the 3D image.

During the labeling of individual vertebrae in the second stage, a combination of L2 loss and cross-entropy loss may be employed. The L2 loss helps refine the continuous voxel-based predictions, while the cross-entropy loss may be used to classify each voxel as belonging to a specific vertebra. This combination supports both accurate localization and categorical labeling of the vertebrae.

For detailed segmentation of individual vertebrae in the third stage, a cross-entropy loss may be applied. Cross-entropy is effective for voxel-wise classification, where each voxel is categorized as either belonging to the vertebra or the background, allowing for precise boundary delineation.

The embodiment of the Spine Segmentation Module 102 where a combination of shape-based and learning-based approaches is used is now described below.

Shape-based segmentation may rely on shape priors or templates that relate to mathematical and/or statistical models representing the typical shape of an object. Common types of shape models include: Statistical shape models, Active shape models, active contour models and deformable shape models, although the skilled person understands that any suitable shape model may additionally or alternatively be used.

Shape-based segmentation may optimize the above models to balance the shape prior and the data fit by using, for example, the equation: E_total = E_shape + lambda*E_data

E_shape encodes the geometric constraints or the deformation allowed in the shape, E_data represents the similarity between the observed image data and the current shape, and lambda weights the importance.

The shape-based model may be initialized at a rough estimate of the object's location in the image. Then, an iterative process may refine the shape to fit the object's boundaries using gradient descent or other numerical optimization methods known to the skilled person. Constraints can also be added to ensure the shape does not deviate significantly from its expected geometry.

When the above is used in combination with learning-based approaches, the segmentation results produced by these shape-based methods can be used to generate additional annotated datasets. For instance, after applying shape-based segmentation to a set of medical images such as, for example, CT or MRI scans, these labeled outputs can serve as a ground truth for training learning models, even when manual annotations are scarce. This is due to the shape-based models needing less data to be trained, i.e., E_data can be down-weighted.

Figure 3 shows a diagram of a method of identifying at least one segment of the at least one segmented vertebra and the determination of suitability for vBMD assessment according to example implementations as described herein.

Figure 3 shows a preferred architecture of the first AI module which identifies the at least one segment of the at least one segmented vertebra and determines the suitability for vBMD assessment of said at least one vertebra based on the identified at least one segment.

The exemplary architecture shown in figure 3 follows a convolutional neural network (CNN) design, with a distinction lying in the structure of the final layers.

The shared encoding backbone 510 of the CNN may function as a generalized feature extractor that is trained to capture common spatial and structural patterns from the data input into said backbone 510. This backbone 510 may consist, preferably, of multiple convolutional and pooling layers that progressively abstract the input image into high-level features relevant to the tasks described below.

However, instead of using a single output head or training separate CNNs for each task, the model described herein may employ multiple task-specific "adapters" 520 in the final layers. These adapters 520 may be fully connected, or may be additional convolutional layers tailored for specific tasks such as, for example, fracture detection, degeneration assessment, malignancy identification, or any other suitable task.

In this multi-task framework, the shared layers may be optimized to learn, and e.g. extract, generalizable features that apply to all tasks, such as, for example, identifying anatomical structures, textures, spatial relationships within the vertebrae, or any other suitable feature.

The task-specific adapters 520 may specialize in analyzing those shared features, and refine them for task-specific predictions. Each adapter 520 preferably operates independently, and may be trained to learn the unique characteristics needed for its respective task such as, for example, fracture morphology and/or signs of degeneration and/or malignancy.

The overall approach is a more efficient and scalable alternative to training individual CNNs for each task, as this approach leverages the shared backbone 510 to process common image features while enabling the adapters 520 to specialize in task-specific decision-making.

The training data for the Vertebra Assessment Module 103 preferably comprises 3D CT image volumes, with (multi-)label annotations at the vertebra level. Each data point, in this example, may include:

1. Fracture: A discrete label, where 0 indicates no fracture and non-zero values represent the fracture grade.
2. Degeneration: A binary label, where 0 denotes no degeneration and 1 indicates a degenerated vertebra.
3. Malignancy: A binary label, with 0 for absence and 1 for presence of malignancy.

Although the skilled person understands that not all of the above annotations are required and that other annotations relating to other characteristics can be used.

For each task-specific adapter 520, a one-hot encoded vector may be generated based on the corresponding label. This vector may then be compared to the ground truth during training using task-appropriate loss functions.

The CNN described above may be trained in a supervised setting, a semi-supervised setting, or an unsupervised setting, as exemplified in the following.

In a fully supervised setting, in this example, the model may be trained based on labeled 3D CT image volumes, wherein each vertebra in the labeled 3D CT image volumes may have multi-label annotations for fracture and/or degeneration and/or malignancy, end-to-end. The process may comprise using a loss function. For each adapter 520, the predicted one-hot vector may be compared to the ground truth labels using appropriate loss functions such as, for example, a cross-entropy loss function for binary labels (degeneration and/or malignancy) and discrete labels (fracture). Gradients may be calculated based on the total loss across all tasks, and the shared CNN backbone 510 and task-specific adapters 520 may be updated using standard stochastic gradient descent (SGD), or an optimizer like, for example, Adam is used.

Alternatively, in the semi-supervised setting, the entire network can be trained even in cases where there are only partial annotations such as, for example, an image has no fracture annotation but has degeneration annotation. In this case, the shared encoder weights and the degeneration adapter weights may be updated while the weights of the adapters 520 whose ground truth is absent may be left untouched. For the unlabeled part of data, model generated predictions can still be made, with these predictions being treated as temporary labels. A consistency loss function may ensure that slight perturbations in input or model parameters do not significantly change predictions.

Lastly, unsupervised learning for pre-training the shared encoder can be used. A possible unsupervised strategy may involve using an autoencoder or a variational autoencoder (VAE). The CNN backbone 510 may extract features, and the model may learn to reconstruct the input CT volumes. The entire autoencoder may be trained, in this example, using a reconstruction loss such as, for example, an L2 distance. Once trained, the decoder may be discarded and the encoder may be attached with the adapters for further training using supervised learning, as mentioned above. Here, the encoder weights can be either frozen or updated with the computed gradients.

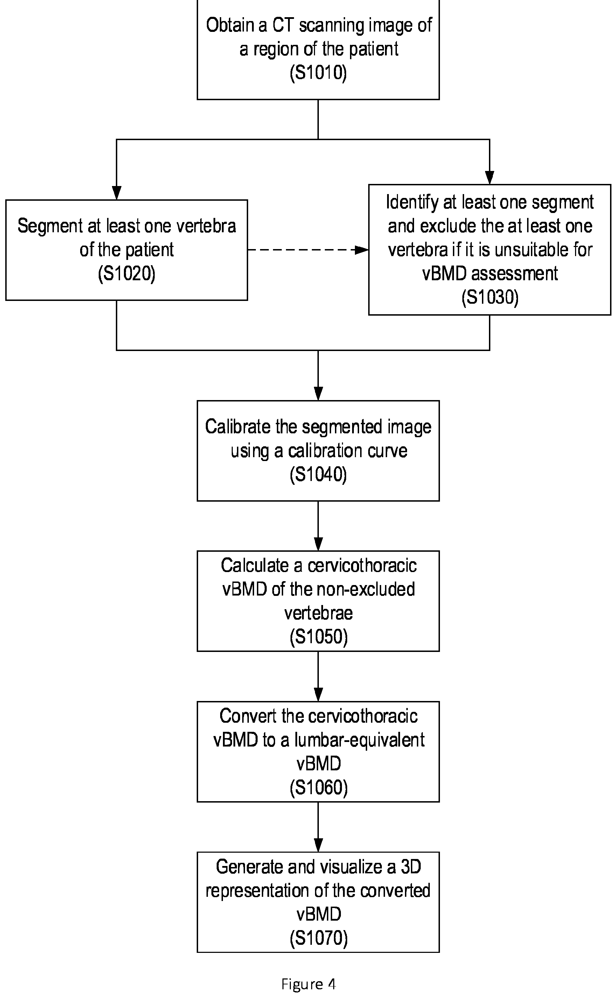

Figure 4 shows a flow chart of a method for visualizing a vBMD of a patient according to the example implementations described herein above.

In the method, the following steps may be undertaken:

- a computed tomography (CT) scanning image of a region of the patient is obtained in step S1010;
- at least one vertebra of the patient located within the obtained image is segmented in step S1020;
- at least one segment of the at least one segmented vertebra is identified by a first AI module, and suitability for vBMD assessment of the at least one segmented vertebra based on the identified at least one segment is determined by the first AI module in step S1030;
- the at least one vertebra from the segmented image if it is determined to be unsuitable for vBMD assessment is then excluded in step S1030;
- optionally, the segmented image may be calibrated in step S1040 using a known calibration curve based on a known metadata parameter of the segmented image;
- a vBMD of a non-excluded at least one segmented vertebra is calculated in step S1050;
- optionally, the calculated vBMD is a cervicothoracic vBMD and is converted to a lumbar-equivalent vBMD based on at least one predetermined conversion factor in step S1060; and
- a three-dimensional representation of the calculated/converted vBMD and visualized on a display in step S1070.

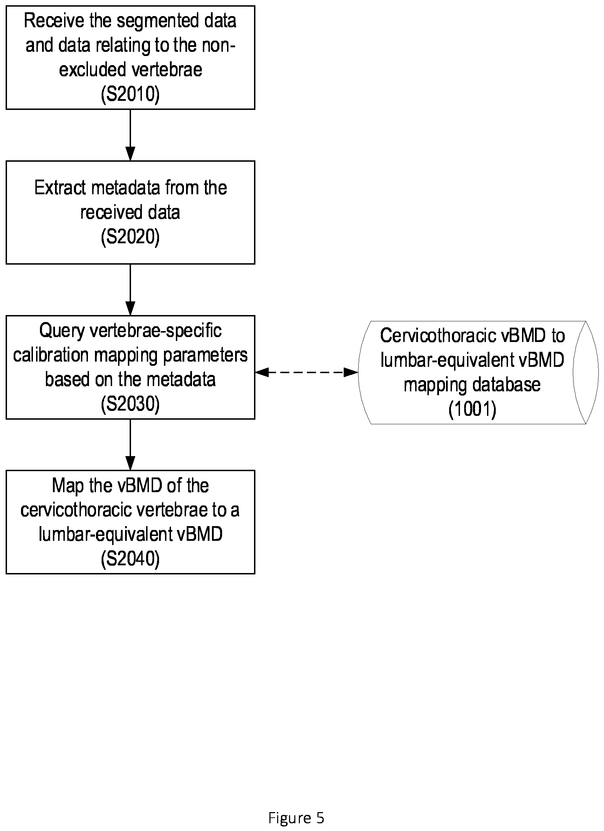

The steps of this method may be undertaken by the respective components of the computing system 10 described above. In some examples, not all of the steps are required. Additionally or alternatively, some steps may be undertaken simultaneously. Figure 5 shows an exemplary flow chart of calculating a cervicothoracic vBMD and converting the cervicothoracic vBMD to a lumbar-equivalent vBMD according to example implementations as described herein.

The method of Figure 5 may be undertaken in the Cervicothoracic BMD Mapping Module 105, as described herein.

In the method for conversion, the Cervicothoracic BMD Mapping Module 105 may receive, in step S2010, the segmented data and data relating to the non-excluded vertebrae from the from the Spine Segmentation Module 102 and/or the Vertebra Assessment Module 103. In some examples, this received data can be calibrated by the CT Calibration Module 104. The Cervicothoracic BMD Mapping Module 105 may then, in step S2020, extract metadata from the data received from the Spine Segmentation Module 102 and/or Vertebra Assessment Module 103. This metadata may comprise at least one of information of the patient from which the CT scanning image was obtained, information on the CT scanner, information relating to the vertebrae not excluded by the Vertebra Assessment Module 103, information on a condition of the segments of the vertebrae segmented by the Spine Segmentation Module 102, or any other suitable metadata such as, for example, scanner metadata relating to the peak voltage (kVp), scanner sequence, scanner manufacturer or make, patient-specific parameters relating to the patient's body composition, table height or contrast stage, and vertebra-specific parameters relating to fractured vertebra, foreign material like metal or cement, or malignant and/or degenerated vertebrae.

Based on this metadata, the Cervicothoracic BMD Mapping Module 105 may query, in step S2030, the vertebrae-specific mapping parameters which have been described in more detail above.

These vertebrae-specific mapping parameters are stored in a mapping database 1001, in this example a cervicothoracic vBMD to lumbar-equivalent vBMD mapping database, but it may additionally or alternatively be any other suitable database, to retrieve the appropriate mapping parameters for the conversion from the cervicothoracic vBMD to the lumbar-equivalent vBMD. Error checks may be performed to ensure the accuracy and integrity of the retrieved parameters, such as verifying consistency with the metadata, extracted from step S2020 and/or the data received from the Spine Segmentation Module 102 and/or Vertebra Assessment Module 103 in step S2010, and validating against predefined acceptable ranges. The verification and/or validation may be based on known ranges of tissue densities within specific regions of interest. For example, for a 120kVp scanner in the (v)BMD space, fat should ideally have a density of -80 $\pm$ 50 HU, and muscle should have ideally a density 30 $\pm$ 50 HU.

The Cervicothoracic BMD Mapping Module 105 may apply the retrieved mapping parameter from the calibration database 1001 to the cervicothoracic vBMD in order to map the cervicothoracic vBMD to a lumbar-equivalent vBMD in step S2040.

The steps of this method may be undertaken by the respective components of the computing system 10 described above. In some examples, not all of the steps are required. Additionally or alternatively, some steps may be undertaken simultaneously.

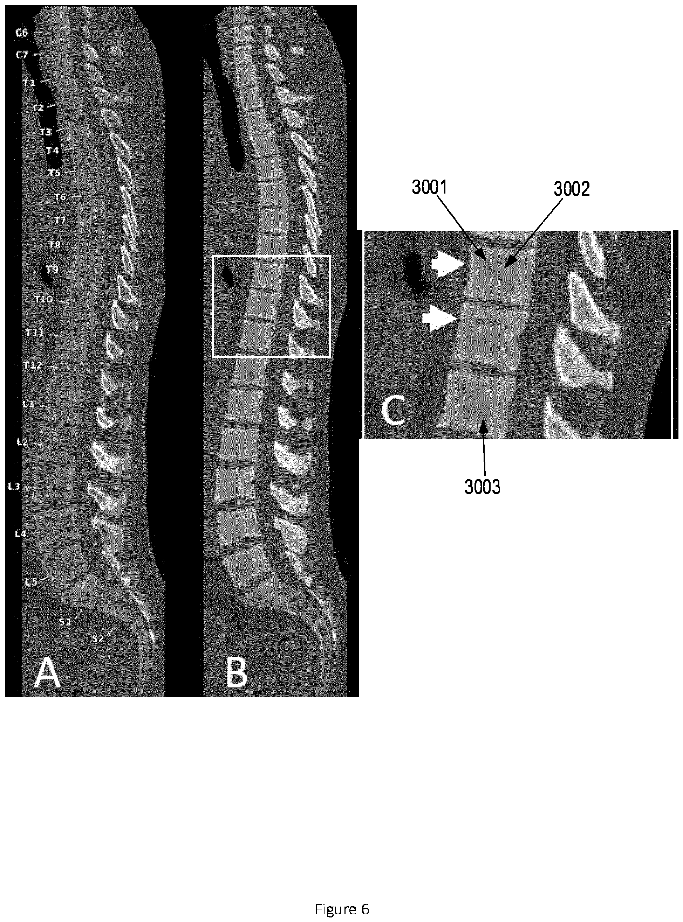

Figure 6 shows an exemplary visual representation of a CT scan with and without the herein-described method according to example implementations as described herein.

In image A of Figure 6, according to the prior art, a sagittal view of a patient's spine, in this example, represented in raw CT data, as would be displayed to a radiologist in, for example, a PACS (Picture Archiving and Communication System) is displayed. Image B depicts the same spine with method as described herein applied, including subregion segmentation of the vertebral body and the trabecular compartment.

In image B, the trabecular compartment is further visualized using a color-coded heatmap representing (v)BMD values, generated from the 3D volumetric image data. Image B is shown in grayscale, but the skilled person understands that this can be converted into any suitable color in such a heat map. In particular, as is seen in more detail in the enlarged portion in image C, the area with the darkest shade, as indicated by arrow 3001, may indicate an abnormal (v)BMD value for that area of the vertebra, the lightest shade, as indicated by arrow 3002, may indicate a border (v)BMD value, and the middle shade, as indicated by arrow 3003, may indicate a normal (v)BMD value. These values may be color coded into red, yellow and green respectively, for example, but the skilled person understands that any suitable color may be used.

With the method as described herein, the radiologist, or other medical professional, is able to better assess: a) the overall distribution of BMD along the spine; and b) focal variations in BMD within single vertebrae. In the example of image C, an early-stage, focal reduction in (v)BMD is observed beneath the upper cortical endplate of vertebrae T9 and T10, whereas vertebra T11 exhibits a normal BMD distribution.

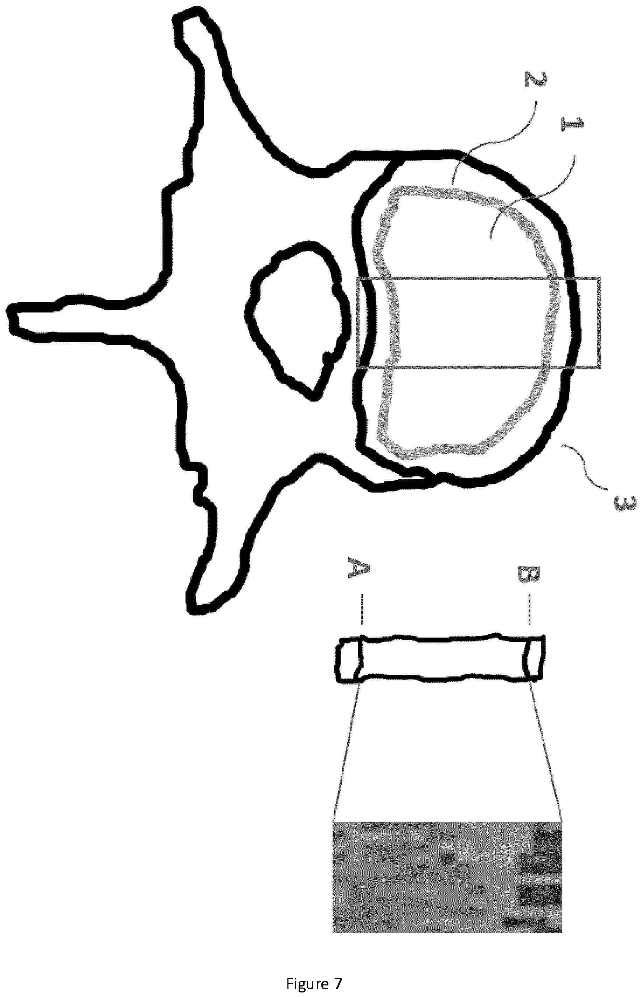

Figure 7 shows a schematic illustration of a vertebra with reference to example implementations as described herein.

Figure 7 shows, in a schematic cross-sectional view of a vertebra, how a trabecular bone mass 1 is surrounded by trabecular bone shell 2, which is then, in turn, surrounded by cortical bone 3. Trabecular bone samples, as seen in the close-up image on the right of the Figure, may be obtained from the entire region reaching from the dorsal (A) to the ventral cortex (B) to obtain a 2D scanning image, alternatively imaged in the craniocaudal direction to obtain a 2D scanning image, or imaged in both directions to obtain a 3D scanning image, although the skilled person understands that any suitable direction(s) may be imaged in order to obtain the 2D or 3D scanning image. The obtained 2D or 3D scanning image may then be used as the CT scanning image obtained by the method (e.g. in line with step S1010 described above). That is to say, this image may then be used as an input for the method described herein.

It is believed that the advantages of the technique presented herein will be fully understood from the foregoing description, and it will be apparent that various changes may be made in the form, constructions and arrangement of the exemplary aspects thereof without departing from the scope of the invention or without sacrificing all of its advantageous effects. Because the technique presented herein can be varied in many ways, it will be recognized that the invention should be limited only by the scope of the claims that follow.

**Claims**

1. A method for generating a three-dimensional visualization for assessing a volumetric bone mineral density, vBMD, of a patient, the method being performed by a computing system and comprising:

   obtaining (S1010) a computed tomography, CT, scanning image of a region of the patient;
   segmenting (S1020) at least one vertebra of the patient located within the obtained image;
   identifying (S1030), by a first artificial intelligence, AI, module, at least one segment of the at least one segmented vertebra and determine suitability for vBMD assessment of the at least one segmented vertebra based on the identified at least one segment;
   excluding (S1030) the at least one vertebra from the segmented image if it is determined to be unsuitable for vBMD assessment;
   calculating (S1050) a vBMD of a non-excluded at least one segmented vertebra;
   generating (S1070) a three-dimensional representation of the calculated vBMD; and
   visualizing (S1070) the generated three-dimensional representation on a display.

2. The method of claim 1, wherein the calculated vBMD is a cervicothoracic vBMD of the non-excluded at least one segmented vertebra,
   wherein the method further comprises:

   converting (S1060) the calculated cervicothoracic vBMD to a lumbar-equivalent vBMD based on at least one predetermined conversion factor,
   wherein the three-dimensional representation is generated from the converted vBMD.

3. The method of claim 1 or 2, wherein the segmentation (S1020) is performed using a shape segmentation method comprising an active shape model and/or a statistical shape model; or

wherein the segmentation (S1020) is performed using a second AI module, wherein the second AI module is trained based on a plurality of CT scanning images different from the obtained scanning image, wherein the plurality of CT scanning images comprise various segmentations of at least one vertebra,

wherein, in particular, segmentation (S1020) is performed on the plurality of CT scanning images based on the shape segmentation method to generate a plurality of labelled segmented scanning images, wherein the second AI module is trained based on the plurality of labelled segmented scanning images.

4. The method of claim 3, wherein the segmentation step (S1020) is configured to output the segmentation as a mask of the segmented image configured to delineate a structure and/or a boundary of the at least one vertebra; and/or wherein the segmentation (S1020) comprises delineation of the at least one vertebra, in particular wherein at least one of the following regions is delineated:

- vertebral body;
- vertebral arch;
- spinous process;
- transverse process;
- superior articular process;
- inferior articular process;
- cortex of the vertebral body; and
- a trabecular component of the vertebral body.

5. The method of any one of the preceding claims, wherein the first AI module is trained to perform the exclusion (S1030) based on a plurality of CT scanning images comprising segmented vertebrae different from the segmented scanning image, wherein a subset of the plurality of CT scanning images comprise at least one vertebra that is considered to be unsuitable for vBMD assessment,

wherein, in particular, a vertebra is determined to be unsuitable for vBMD assessment in the case of the presence of at least one of:

- an osteophyte formation on the at least one vertebra;
- a sclerotic lesion on the at least one vertebra;
- a fracture of the at least one vertebra;
- a metal implant in the at least one vertebra; and
- cement in and/or on the at least one vertebra.

6. The method of claim 5, wherein the first AI module is a neural network and comprises at least one shared layer configured to extract common features from the segmented image, and at least one task-specific adapter in a layer subsequent to the at least one shared layer, wherein the at least one task-specific adapter is configured to analyze task-specific features, wherein, in particular:

(a) the task-specific features are associated with at least one of the following tasks:

- fracture detection,
- degeneration assessment, and
- malignancy identification; and/or

(b) the first AI module is trained based on a plurality of segmented CT scanning images different from the segmented CT scanning image output in the segmentation step (S1020), and is trained to output an indication that the at least one vertebra from the segmented image is unsuitable for vBMD assessment based on at least one of the task-specific features.

7. The method of claim 6, wherein the extracted common features from the segmented image relate to the at least one task-specific adapter, in particular wherein the common features are associated with at least one of:

- an anatomical structure;
- a texture of the segmented image; and
- a spatial relationship within the at least one vertebra.

8. The method of claim 6 or 7, wherein, the at least one task-specific adapter is trained based on the extracted common

# EP 4 717 181 A1

features output from the at least one shared layer to extract a characteristic of the at least one vertebra related to its specific task, and is trained to output the indication that the at least one vertebra from the segmented image is unsuitable for vBMD assessment based on the extracted characteristic.

9. The method of any one of the preceding claims, wherein the method further comprises:

calibrating (S1040) the segmented image using a known calibration curve based on a known metadata parameter of the segmented image, wherein, in particular, the calibration curve is a linear calibration curve which comprises a known calibration slope and intercept based on the known metadata parameter,
wherein, in particular, the computing system uses a database query module and a conversion module, wherein:

the database query module is configured to communicate with a calibration database to retrieve a relevant calibration parameter based on the known metadata parameter;
the conversion module is configured to generate the linear calibration curve based on the retrieved calibration parameter; and
wherein the calibration step further comprises applying the linear calibration curve to the segmented image.

10. The method of any one of claims 2 to 9, wherein the conversion step (S1060) further comprises:

retrieving, from a database, at least one mapping parameter reflecting the at least one predetermined conversion factor, wherein the database comprises information relating to a relationship between a vBMD value of a cervicothoracic vertebra and a corresponding lumbar-equivalent vBMD value; and
performing the conversion step based on the retrieved at least one mapping parameter
wherein, in particular, the at least one mapping parameter is specific to a population-based equation factor, wherein, in particular, the at least one population-based equation factor is based on at least one of:

- a cervicothoracic vertebra level of the patient;
- an age of the patient; and
- a gender of the patient.

11. The method of any one of the preceding claims, wherein the three-dimensional representation of the calculated vBMD comprises a color-coded heat map of the calculated vBMD, wherein, in particular, at least one color of the color-coded heat map is based on patient information and/or a clinical guideline.

12. The method of any one of the preceding claims, wherein the method is configured to visualize (S1070) a vBMD of an entire spine of the patient.

13. A computing system comprising means for carrying out the method of any one of the preceding claims.

14. A computer program product comprising instructions which, when the program is executed by a computing system, cause the computing system to carry out the method of any one of claims 1 to 12.

15. A computer-readable medium having stored thereon the computer program product of claim 14.

10

Computing system

Processor 12

Memory 14

Figure 1a

10

CT image acquisition module

101

Spine segmentation module

102

Vertebrae assessment module

103

CT image calibration module
104

| Database query module 104a | HU-BMD conversion module 104b |
|---|---|

Cervicothoracic BMD mapping module

105

3D visualization module

106

Figure 1b

*Input:* CT Scan → Detection Net → Detection heat-map → Spine bounding box

Sagittal & Coronal MIPs → Labelling Net → 2D Vertebral labels → 3D Vertebral labels

3D Vertebral Patch / Gaussian Patch → Segmentation Net → 3D Vertebral Mask → *Output:* Multi-label Segmentation Mask

Figure 2

Fracture
detecting
adapter

Malignancy
detecting
adapter

Degeneration
detecting
adapter

Feature extracting CNN
backbone

Multi-task adapters

510

520

Figure 3

EP 4 717 181 A1

Figure 4

22

Figure 5

Figure 6

Figure 7

Europäisches
Patentamt

European
Patent Office

Office européen
des brevets

# EUROPEAN SEARCH REPORT

**Application Number**

EP 24 20 3514

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2015/348259 A1 (SOUZA ANDRE [US] ET AL) 3 December 2015 (2015-12-03) | 1-5,9-15 | INV. A61B6/46 |
| A | * abstract * <br> * paragraph [0002] * <br> * paragraph [0013] - paragraph [0016] * <br> * paragraph [0043] - paragraph [0051] * <br> * paragraph [0060] * <br> * figures 5A, 5B, 8 * | 6-8 | |
| Y | RÜHLING SEBASTIAN ET AL: "Proposed diagnostic volumetric bone mineral density thresholds for osteoporosis and osteopenia at the cervicothoracic spine in correlation to the lumbar spine", EUROPEAN RADIOLOGY ;, vol. 32, no. 9, 6 April 2022 (2022-04-06), pages 6207-6214, XP093256080, Berlin, DE ISSN: 1432-1084, DOI: 10.1007/s00330-022-08721-7 Retrieved from the Internet: URL:https://link.springer.com/article/10.1 007/s00330-022-08721-7/fulltext.html> | 1-5,9-15 | |
| A | * abstract * <br> * section "Evaluation of vertebrae and vBMD extraction" * <br> * section "CT imaging and data processing" * <br> * section "Statistical analysis" * <br> * figures 1,3,5 * <br> * table 2 * | 6-8 | **TECHNICAL FIELDS SEARCHED (IPC)** <br><br> A61B |

-----

-/--

The present search report has been drawn up for all claims

| Place of search | Date of completion of the search | Examiner |
|---|---|---|
| Munich | 6 March 2025 | Montes, Pau |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.......................................................................................

& : member of the same patent family, corresponding document

EPO FORM 1503 03.82 (P04C01)

Europäisches
Patentamt

European
Patent Office

Office européen
des brevets

**EUROPEAN SEARCH REPORT**

**Application Number**

EP 24 20 3514

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GUINEBERT SYLVAIN ET AL: "Automatic semantic segmentation and detection of vertebras and intervertebral discs by neural networks", COMPUTER METHODS AND PROGRAMS IN BIOMEDICINE UPDATE, vol. 2, 1 January 2022 (2022-01-01), page 100055, XP093256389, ISSN: 2666-9900, DOI: 10.1016/j.cmpbup.2022.100055 * abstract * * section "Materials and Methods" * ----- | 1-15 | |
| A | HANS LIEBL ET AL: "A Computed Tomography Vertebral Segmentation Dataset with Anatomical Variations and Multi-Vendor Scanner Data", ARXIV.ORG, CORNELL UNIVERSITY LIBRARY, 201 OLIN LIBRARY CORNELL UNIVERSITY ITHACA, NY 14853, 10 March 2021 (2021-03-10), XP081909272, * abstract * * section "Abstract & Summary" * ----- | 1-15 | **TECHNICAL FIELDS SEARCHED (IPC)** |
| A | SAEED MUHAMMAD USMAN ET AL: "An Automated Deep Learning Approach for Spine Segmentation and Vertebrae Recognition Using Computed Tomography Images", DIAGNOSTICS, vol. 13, no. 16, 12 August 2023 (2023-08-12), page 2658, XP093256212, CH ISSN: 2075-4418, DOI: 10.3390/diagnostics13162658 * abstract * * section "Materials and Methods" * ----- | 1-15 | |

The present search report has been drawn up for all claims

| Place of search | Date of completion of the search | Examiner |
|---|---|---|
| Munich | 6 March 2025 | Montes, Pau |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

                              

& : member of the same patent family, corresponding
   document

EPO FORM 1503 03.82 (P04C01)

page 2 of 2

## ANNEX TO THE EUROPEAN SEARCH REPORT
## ON EUROPEAN PATENT APPLICATION NO.

EP 24 20 3514

This annex lists the patent family members relating to the patent documents cited in the above-mentioned European search report.
The members are as contained in the European Patent Office EDP file on
The European Patent Office is in no way liable for these particulars which are merely given for the purpose of information.

06-03-2025

| Patent document cited in search report | Publication date | Patent family member(s) | Publication date |
|---|---|---|---|
| US 2015348259 A1 | 03-12-2015 | NONE | |

EPO FORM P0459

For more details about this annex : see Official Journal of the European Patent Office, No. 12/82

**EP 4 717 181 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150348259 A1
- EP 24203433